# EUROPÄISCHE PATENTSCHRIFT

(11) **EP 2 408 410 B1**
(45) Veröffentlichungstag und Bekanntmachung des Hinweises auf die Patenterteilung: **20.09.2017**
(21) Anmeldenummer: 09776446.8
(22) Anmeldetag: 17.03.2009
(51) Int. Cl.: A61F 9/008, A61B 18/20, A61B 18/22, A61F 9/009

(54) **LASEREINRICHTUNG FÜR DIE OPHTHALMOLOGIE**
LASER DEVICE FOR OPHTHALMOLOGY
DISPOSITIF LASER POUR L'OPHTALMOLOGIE

(43) Veröffentlichungstag der Anmeldung: 25.01.2012
(73) Patentinhaber: WaveLight GmbH, 91058 Erlangen (DE)
(72) Erfinder: VOGLER, Klaus, 99444 Blankenhain (DE); DONITZKY, Christof, 90542 Eckental/Eschenau (DE); KITTELMANN, Olaf, 14109 Berlin (DE)
(74) Vertreter: Katérle, Axel
(86) Internationale Anmeldenummer: PCT/EP2009/001958
(87) Internationale Veröffentlichungsnummer: WO 2010/105637

(56) Entgegenhaltungen:
- EP-A- 1 792 593
- EP-A1- 1 486 185
- DE-A1- 10 206 663
- US-A1- 2005 259 933
- US-A1- 2007 027 439

## Beschreibung

Die Erfindung betrifft eine Lasereinrichtung für die Ophthalmologie.

In der refraktiven ophthalmologischen Chirurgie werden durch Eingriffe am Auge eines Patienten die Brechungseigenschaften des Auges zur Korrektur von Sehfehlern verändert. Große Bedeutung hat dabei das sogenannte LASIK-Verfahren (LASer-In-Situ-Keratomileusis), bei dem zunächst ein flacher Hornhautschnitt gesetzt wird, durch den ein Deckelscheibchen - der sogenannte "Flap" - entsteht. Dieser kann zur Seite geklappt werden, um das darunter liegende Hornhautgewebe (Stroma) freizulegen. Anschließend wird mit einem Laser (regelmäßig einem Excimer-Laser) Stromagewebe nach Maßgabe eines patientenindividuell ermittelten Ablationsprofils abgetragen (ablatiert). Danach wird der Flap zurückgeklappt; die Wunde verheilt relativ schnell.

Die Photoablation von Korneagewebe beruht auf der durch Absorption der Strahlungsenergie bewirkten Zerstörung des Gewebematerials. Die für die Ablation eingesetzte Strahlungswellenlänge muss unterhalb der Grenze liegen, ab der das Korneagewebe transmissiv wird. Diese Grenze liegt beim Menschen bei etwa 300 nm. Die gängige Alationswellenlänge sind die 193 nm des ArF-Excimer-Lasers. Die pro Puls bewirkte Ablationsdicke ist äußerst gering und liegt beispielsweise im Bereich von Zehntel Mikrometern.

Zur Anbringung des Flapschnitts bei der LASIK wurde in jüngerer Zeit das früher verwendete mechanische Mikrokeratom durch einen Fs-Laser ersetzt, also einen Laser, der gepulste Laserstrahlung mit Pulsdauern im Femtosekundenbereich erzeugt. Für einen intrageweblichen Schnitt muss die Laserstrahlung im transmissiven Wellenlängenbereich der Kornea liegen, also über etwa 300 nm. Zugleich muss die Leistungsdichte im Strahlfokus groß genug, um einen optischen Durchbruch zu erzeugen, die sogenannte Photodisruption. Deren Wirkbereich ist lokal auf den Fokusdurchmesser beschränkt. Um einen flächigen Schnitt zu erzeugen, muss deshalb der Strahlfokus nacheinander oder nach einem anderen Abtastmuster auf eine Vielzahl eng benachbarter Punkte in der gewünschten Schnittfläche (-ebene) bewegt werden.

Gegenwärtig werden für die Korrektur von Fehlsichtigkeiten Gerätesysteme mit einem Excimer-Laser eingesetzt, wobei weitere separate Einzelgeräte assistieren, etwa zur Diagnostik, zur Vermessung oder zur Anbringung von Schnitten. Alle diese Zusatzgeräte müssen im Umfeld des Excimer-Lasers platziert werden, um die Operation zügig und ohne komplizierte Umlagerungen des Patienten ausführen zu können.

Die bisherige Praxis in Kliniken und Arztpraxen sieht üblicherweise die Verwendung zweier getrennter Lasersysteme vor, eines Excimer-Lasers für die Ablation und eines Fs-Lasers für die Anbringung von Inzisionen. Die beiden Lasersysteme werden dabei nahe beieinander im selben Raum aufgestellt und sind beispielsweise durch eine schwenkbare oder verschiebbare Patientenliege erreichbar, die es gestattet, den Patienten von einem System unter das andere zu bewegen. Dies macht die Durchführung einer aus einer Laserinzision und einer Photoablation bestehenden Operation insofern für den Patienten angenehm, als er sich nur einmal hinlegen muss und sich nicht aktiv zwischen den Teiloperationen bewegen oder gar selbst aufstehen muss.

Als Alternative zu einer Bewegung der Patientenliege ist im Stand der Technik eine Lösung bekannt geworden, bei der das Fs-Lasersystem mit einem beweglichen Spiegelgelenkarm ausgestattet ist. Der Patient wird dabei mit seinem zu behandelnden Auge unter dem Strahlaustrittsfenster des Ablationslasersystems positioniert. Der Spiegelgelenkarm kann in den Bereich zwischen Auge und Strahlaustrittsfenster hineingeschwenkt werden, um auf diese Weise beispielsweise bei der LASIK den initialen Flapschnitt vornehmen zu können. Anschließend wird der Spiegelgelenkarm zurückgeschwenkt, so dass das Auge frei ist für die Behandlung mit dem Ablationslaserstrahl. Nachteilig an solchen Lösungen mit Spiegelgelenkarm ist die begrenzte Genauigkeit bei der Laserstrahlführung, da sich in jeder Lage des Arms unterschiedliche Toleranzketten aus der Präzision der Justage der einzelnen Spiegel ergeben.

Zur Verkleinerung eines sowohl für die korneale Ablation als auch für intrakorneale Schnitte geeigneten Gerätesystems könnte man daran denken, die für die Ablation benötigte UV-Strahlung und die für den kornealen Schnitt benötigte Fs-Strahlung aus ein und derselben Laserstrahlungsquelle zu beziehen, so dass man mit einer einzigen Quelle auskommt. Beispielsweise könnte die UV-Strahlung durch Frequenzwandlung aus einer IR-Grundwelle erzeugt werden. Die hierfür benötigten Frequenzwandler weisen jedoch eine relativ große Instabilität und Unzuverlässigkeit auf, insbesondere bei Wandlung auf Wellenlängen im UV-Bereich unterhalb von 300 nm, und zwar aufgrund der inhärenten Kompromisse, die eingegangen werden müssen, um zwei oder mehr Wellenlängen mit den für die Applikation ausreichenden Zielparametern zu realisieren. Jede Einzelwellenlänge kann nur suboptimal erzeugt werden. Somit ist in den meisten Fällen der summarische Wirkungsgrad des Gesamtgeräts schlecht und es muss verhältnismäßig viel elektrische Energie eingespeist werden. Die Kühlung wird dadurch aufwendig und das Gerät wird entsprechend groß. Somit wird auch der Vorteil, anstatt zweier Geräte nur ein einziges zu benötigen, stark geschmälert.

Weiterhin sind im Stand der Technik Methoden bekannt geworden, die es gestatten, allein mit einem Fs-Laser refraktive Behandlungen des Auges durchzuführen. Ein diesbezügliches Beispiel ist die korneale Lentikelextraktion, bei der mittels des Fs-Lasers ein vollständig innerhalb des Korneagewebes liegendes Lentikel (linsenförmiges Scheibchen) freigeschnitten wird, das anschließend durch einen seitlichen Einschnitt in der Kornea herausgeholt wird. Auch dieser seitliche Einschnitt kann gewünschtenfalls mit dem Fs-Laser angebracht werden. Ein zusätzlicher Excimer-Laser für die Ablation ist bei diesen Lösungen nicht nötig. Weil beim laserinduzierten optischen Durchbruch jedoch die Fokusabmessungen die Größe der Disruption bestimmen, ist bei rein Fs-basierten refraktiven Behandlungsmethoden zumindest derzeit nicht die gleiche hohe Korrekturgenauigkeit wie bei der Ablation erreichbar. werden.

Aufgabe der Erfindung ist es, eine sowohl für die korneale Ablation als auch für Inzisionen geeignete Lasereinrichtung für die Ophthalmologie zu schaffen, die als vergleichsweise kompaktes Gerät ausführbar ist, möglichst wenig relative Umpositionierungen zwischen Patient und Gerät während einer Operation erfordert und eine sowohl ablatierende als auch schneidende Laserbehandlung mit jeweils optimal angepassten Strahlungsparametern gestattet.

Zur Lösung dieser Aufgabe sieht die Erfindung eine Lasereinrichtung für die Ophthalmologie vor, umfassend Komponenten zur Bereitstellung eines ersten gepulsten Laserstrahls mit auf die Ablation von Hornhautmaterial abgestimmten Strahleigenschaften und eines zweiten gepulsten Laserstrahls mit auf die Anbringung einer Inzision im Augengewebe abgestimmten Strahleigenschaften, wobei die Komponenten gesonderte Laserquellen zur Erzeugung der beiden Laserstrahlen sowie eine Vielzahl optischer Elemente umfassen, welche die beiden Laserstrahlen auf gesonderten Strahlwegen zu einem jeweiligen Strahlaustrittsort führen und auf einen außerhalb des Strahlaustrittsorts liegenden Brennpunkt fokussieren. Als Stand der Technik hierzu wird die EP1792593 angesehen. Erfindungsgemäß umfassen die optischen Elemente einen der Führung des zweiten Laserstrahls dienenden Lichtwellenleiter, wobei zumindest die beiden Laserquellen in einem gemeinsamen Gehäuse untergebracht sind und der Lichtwellenleiter zumindest auf einem Teil seiner Länge innerhalb des Gehäuses verläuft.

Durch die Separierung der Strahlwege der beiden Laserstrahlen und die gleichzeitige Realisierung zumindest eines Teils des Strahlwegs des zweiten Laserstrahls durch einen Lichtwellenleiter ist ein hoher Integrationsgrad erzielbar. Dies gestattet eine vergleichsweise kleinvolumige Bauweise. Die Vorsehung gesonderter Strahlwege für die beiden Laserstrahlen gestattet es darüber hinaus, für jeden Strahlweg ein auf die Eigenschaften der Strahlemission hin optimiertes Strahlführungskonzept zu entwickeln und einzusetzen. Es müssen keine komplizierten, beispielsweise dichroitischen oder mit doppelten HR-Beschichtungen (HR: Highly Reflective) versehenen Optiken zur Führung von Strahlung aus völlig verschiedenen Spektralbereichen verwendet werden, wie es beispielsweise bei Lösungen mit einer gemeinsamen Strahlungsquelle und zumindest teilweise gemeinsamem Strahlweg notwendig ist. Durch die Verwendung zweier separater Laserquellen kann die für den jeweiligen gewünschten Behandlungszweck optimale Strahlqualität ohne erhöhten Energie-/Kühlungsaufwand bereitgestellt werden. Je nach Anwendungsfall oder Integrationsgrad kann zudem die Abgabe des zweiten Laserstrahls an einer ortsfesten Stelle der Lasereinrichtung erfolgen oder mit Hilfe des Lichtwellenleiters flexibel an verschiedene Orte bewegbar sein. Insbesondere die Verwendung eines Lichtwellenleiters erlaubt eine einfache und platzsparende Integration der Laserquelle für den zweiten Laserstrahl in die Lasereinrichtung.

Gemäß einer bevorzugten Ausführungsform ist die erste Laserquelle ein Excimer-Laser und die zweite Laserquelle ein Faserlaser. Faserlaser sind in der Lage, eine vergleichsweise hohe Strahlqualität (typischerweise mit einem Strahlparameterprodukt M² ≤ 1,3) bei sehr kompakter Bauweise zu bieten. Der hohe Wirkungsgrad von Faserlasern, der beispielsweise bei ungefähr 30% liegt, erhöht den elektrischen Leistungsbedarf des Gesamtgeräts nur unwesentlich. Beispielsweise kann sich der Leistungsbedarf durch den Faserlaser um nur sowenig wie höchstens 150 Watt gegenüber einer Lasereinrichtung erhöhen, die nur einen Ablationslaser besitzt. Die erfindungsgemäße Anordnungsweise der beiden Laserquellen in dem Gehäuse sieht vor, dass die zweite Laserquelle in dem Gehäuse unterhalb der ersten Laserquelle angeordnet ist, wobei der Lichtwellenleiter innerhalb des Gehäuses von der zweiten Laserquelle seitlich an der ersten Laserquelle vorbei nach oben geführt ist.

Das Gehäuse kann einen zur Aufstellung auf einer horizontalen Aufstellfläche (z.B. Fußboden) vorgesehenen, einen Boden des Gehäuses umfassenden Basisteil sowie einen in vertikalem Abstand vom Gehäuseboden quer von dem Basisteil abstehenden Gehäusearm aufweisen. Dabei ist es bevorzugt, dass die beiden Laserquellen zumindest größtenteils in dem Basisteil des Gehäuses untergebracht sind und zumindest ein Teil der optischen Elemente in dem Gehäusearm untergebracht ist. Die Strahlwege der beiden Laserstrahlen verlaufen dementsprechend zumindest teilweise durch den Gehäusearm. Für eine möglichst geringe Aufstandsfläche des Gehäuses empfiehlt es sich, die beiden Laserquellen übereinander anzuordnen, wozu in dem Basisteil des Gehäuses beispielsweise ein oder mehrere Zwischenböden vorhanden sein können, auf welche die verschiedenen Laserquellen gestellt werden können.

In den Gehäusearm ist vorzugsweise ein den ersten Laserstrahl führendes, zweckmäßigerweise mit einem Inertgas (z.B. Stickstoff) gespültes erstes Strahlrohr eingebaut, das unter anderem einen Strahlhomogenisierer, einen Scanner sowie mindestens eine Fokussierlinse zur Fokussierung des ersten Laserstrahls auf einen außerhalb des Gehäuses liegenden Brennpunkt enthalten kann. Zumindest ein Teil des Strahlwegs des zweiten Laserstrahls verläuft dabei in dem Gehäusearm außerhalb des ersten Strahlrohrs. Außerdem ist an dem Gehäusearm ein Austrittsfenster für den ersten Laserstrahl gebildet.

Gemäß einer Ausführungsform kann in dem Gehäusearm ein längs des ersten Strahlrohrs verlaufendes zweites Strahlrohr eingebaut sein, durch welches der Strahlweg des zweiten Laserstrahls verläuft. Der Lichtwellenleiter erstreckt sich dabei innerhalb des Gehäuses zwischen der zweiten Laserquelle und dem zweiten Strahlrohr.

Der Gehäusearm kann in Armlängsrichtung im Abstand vor oder hinter dem Austrittsfenster für den ersten Laserstrahl eine Fokussieroptik für den zweiten Laserstrahl tragen. Bevorzugt ist die Fokussieroptik als F-Theta-Objektiv ausgeführt.

Gemäß einer Variante kann der Lichtwellenleiter aus dem Gehäuse herausgeführt sein und in einem zumindest mit einem Scanner und einer Fokussieroptik ausgestatteten Handstück zur Applikation des zweiten Laserstrahls enden. Das Handstück ist dabei zweckmäßigerweise mit dem Gehäuse allein durch ein oder mehrere flexible Kabel verbunden, von denen eines den Lichtwellenleiter enthält. In dem mindestens einen Kabel können neben dem Lichtwellenleiter beispielsweise noch eine elektrische Stromzufuhr, eine Saugleitung sowie eine oder mehrere Datenleitungen verlaufen. Das Handstück selbst ist im Rahmen der Beweglichkeit des mindestens einen Kabels unabhängig von dem Gehäuse und den darin oder daran angebrachten Komponenten der erfindungsgemäßen Lasereinrichtung positionierbar. Größe und Form des Handstücks sind zweckmäßigerweise so, dass das Handstück bequem mit einer Hand gegriffen und an das zu behandelnde Auge herangeführt werden kann, um dort mit dem über das Handstück abgegebenen zweiten Laserstrahl eine Inzision im Augengewebe anzubringen.

Der Lichtwellenleiter kann aus dem Gehäuse an einer Stelle herausgeführt sein, die an dem Gehäusearm in Armlängsrichtung im Abstand vor oder hinter dem Austrittsfenster für den ersten Laserstrahl liegt.

Neben einem Scanner und einer Fokussieroptik kann das Handstück zudem einen Pulskompressor zur zeitlichen Kompression der Pulse des zweiten Laserstrahls enthalten, wobei der Pulskompressor vorzugsweise ein optisches Miniaturgitter aufweist, etwa ein Transmissionsgitter oder eine photonische Kristallfaser (PCF: Photonic Crystal Fiber).

Das Handstück kann mit Koppelformationen ausgeführt sein, die eine mechanische Ankopplung des Handstücks an ein zu behandelndes Auge oder einen auf das Auge aufgesetzten Saugring gestatten. Auf diese Weise ist eine definierte Referenzierung des Fokusorts des zweiten Laserstrahls in Strahlausbreitungsrichtung gegenüber dem zu behandelnden Auge erzielbar. Die Koppelformationen können beispielsweise einen fest oder lösbar an dem Handstück angebrachten Adapter umfassen, der in gegebenenfalls saugkraftunterstützte Koppelanlage mit einem bereits zuvor auf dem Auge platzierten Saugring bringbar ist.

Der in dem Handstück vorgesehene Scanner ist beispielsweise von einem elektrooptischen Kristall gebildet, mit dem sich der zweite Laserstrahl räumlich steuern lässt. Derartige elektrooptische Kristalle basieren üblicherweise auf dem Pockels- oder Kerr-Effekt, bei dem durch Anlegen eines elektrischen Felds an das Kristall dessen optische Eigenschaften, wie beispielsweise die Brechzahl, verändert werden. Auch akusto-optische Modulatoren können durch das induzierte Bragg-Gitter schnelle steuerbare Strahlablenkungen bewirken. Alternativ kann der in dem Handstück vorhandene Scanner beispielsweise ein elektrooptisches Hologramm sein, das durch Aufzeichnen eines Volumenphasenhologramms in einer flüssigen Kristallmonomermischung hergestellt wird und durch externe elektrische Spannungen effiziente und kontrollierbare Strahlauslenkungen erzeugt.

Hingegen dient zur Abtastung des ersten Laserstrahls vorzugsweise ein nach dem Galvanometerprinzip arbeitender Spiegelscanner mit zwei um zueinander senkrechte Achsen kippbar angeordneten Spiegeln. Es versteht sich jedoch, dass auch für die Abtastung des ersten Laserstrahls gewünschtenfalls andere Abtastprinzipien zum Einsatz kommen können.

Die von der zweiten Laserquelle erzeugten Laserpulse besitzen bevorzugt eine Pulsdauer im Femtosekundenbereich, wobei zwischen die zweite Laserquelle und den Lichtwellenleiter ein Pulsstrecker zur zeitlichen Streckung der Laserpulse auf Pulslängen größer als eine Pikosekunde geschaltet sein kann. Die Pulsstreckung erlaubt eine Herabsetzung der Intensität der Laserpulse. Dies führt wiederum zu einer geringeren Belastung des Lichtwellenleiters.

Der Lichtwellenleiter kann beispielsweise eine photonische Kristallfaser (PCF-Faser) oder eine Transmissionsfaser mit einem großen Modenfeld sein. Geeignete LMA-Fasern besitzen beispielsweise einen Kerndurchmesser von 20 µm bis über 40 µm. Aufgrund der Verteilung der Lichtleistung auf eine größere Fläche und der gleichzeitigen Weiterleitung in niedriger Modenordnung bzw. in der Grundmode erlauben LMA-Fasern eine Transmission der von der zweiten Laserquelle abgegebenen Strahlung, ohne die Strahl parameter zu verschlechtern oder die LMA-Faser durch zu hohe Intensitäten zu zerstören. PCF-Fasern erlauben durch große Kerndurchmesser oder bei Ausbildung als Hohlleiter ebenfalls hohe Leistungstransmissionen. Sie sind ein anderer Fasertyp als LMA-Fasern und beruhen nicht auf Totalreflexion, sondern auf dem photonischen Bandgap-Effekt.

Es kann von Vorteil sein, wenn zumindest ein Abschnitt des Lichtwellenleiters eine zeitliche Pulskompression der Laserpulse des zweiten Laserstrahls bewirkt. Dies ermöglicht einen noch kompakteren Aufbau der Lasereinrichtung als mit Standard-Kompressionsgittern. Die ansonsten in einem separaten Bauteil (z.B. einem Kompressionsgitter) durchzuführende Kompression der Laserpulse kann somit auf den flexiblen Lichtwellenleiter verlagert werden; Kompressionskomponenten außerhalb des Lichtwellenleiters können demnach entfallen.

Eine diesbezüglich besonders bevorzugte Ausführungsform sieht vor, dass zumindest der die Pulskompression bewirkende Abschnitt des Lichtwellenleiters von einer photonischen Hohlkernfaser gebildet ist. Dieser Typ von PCF-Faser bezeichnet eine mikrostrukturierte optische Faser, die im Kern- oder im Mantelbereich typischerweise feine, mit Luft oder einem Gas gefüllte Kapillarstrukturen enthält. Durch Variation der Lochmittenabstände und der Durchmesser der Kapillarstrukturen können die optischen Parameter der Faser und die Eigenschaften der Lichtführung gesteuert werden. Insbesondere kann so eine Pulskompression der Laserpulse des zweiten Laserstrahls erreicht werden.

Die Erfindung gestattet die Integration eines Fs-Lasers für die Erzeugung einer intrageweblichen Inzision und eines Excimer-Lasers für die Hornhautablation in einem Gesamtgerät, wobei die beiden Laser mit ihren für die jeweilige Applikation optimalen Parametern betrieben werden können. Es sind keine Kompromisse oder zueinander gegensätzliche Optimierungen notwendig, wie sie bei Lösungen mit Frequenzwandlern, die aus einer einzigen Laserstrahlungsquelle verschiedene Wellenlängen erzeugen, unvermeidbar sind. Daher gestattet es die erfindungsgemäße Lasereinrichtung, gleichermaßen optimale Behandlungsergebnisse zu erzielen, wie sie mit speziell zugeschnittenen Einzelgeräten möglich sind. Die Verwendung eines Faserlasers für die zweite Laserquelle erlaubt eine einfache Integration ohne wesentlichen zusätzlichen Platzbedarf. Die Verwendung eines flexiblen Lichtwellenleiters zur Führung des von dem Faserlaser erzeugten Laserstrahls bietet eine hohe gestalterische Flexibilität bei der Unterbringung der Komponenten der erfindungsgemäßen Lasereinrichtung in einem Gehäuse.

Bei einer Ausführungsform der Laserseinrichtung, bei der der zweite Laserstrahl über ein Handstück applizierbar ist, erfolgt die Führung des zweiten Laserstrahls zweckmäßigerweise auf der gesamten Strecke von der zweiten Laserquelle bis zu dem Handstück über einen flexiblen Lichtwellenleiter. In dem Handstück oder schon zuvor entlang des Lichtwellenleiters erfolgt dann eine zeitliche Pulskompression der vor Einspeisung in den Lichtwellenleiter in den Pikosekundenbereich gestreckten, ursprünglich eine Pulsdauer im fs-Bereich besitzenden Laserpulse. Außerdem erfolgt in dem Handstück zweckmäßigerweise eine zweidimensionale Ablenkung des zweiten Laserstrahls mit Hilfe geeigneter optischer Miniaturbauelemente. Das Handstück, das man auch als Handapplikator bezeichnen kann, kann durch seine spezielle Bauform an die konkrete Anwendung angepasst sein. Insbesondere ist es vorstellbar, wechselbare Handstücke oder Handstückeinsätze zu realisieren, die beispielsweise über eine mechanisch lösbare Schnittstelle an ein den Lichtwellenleiter enthaltendes Kabel anschließbar sein können. Damit kann die erfindungsgemäße Lasereinrichtung für weitere, über den Flapschnitt bei der LASIK hinausgehende Fs-Laserapplikationen am Auge eingesetzt werden.

Die Erfindung wird nachfolgend anhand der beigefügten Zeichnungen näher erläutert. Es stellen dar:
- Fig. 1: schematisch ein erstes Ausführungsbeispiel einer erfindungsgemäßen Lasereinrichtung und
- Fig. 2: schematisch ein zweites Ausführungsbeispiel einer erfindungsgemäßen Lasereinrichtung.

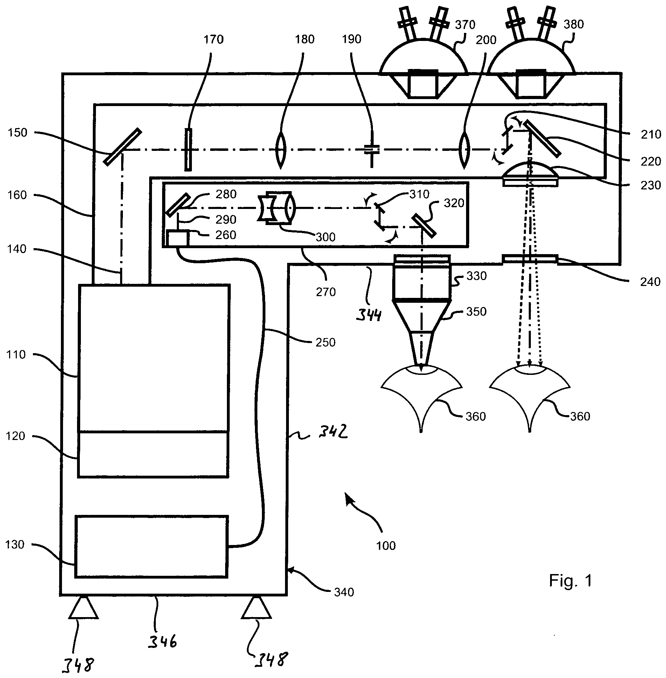

Die Lasereinrichtung gemäß dem in Fig. 1 gezeigten Ausführungsbeispiel ist als integrales opthalmologisches Laserchirurgiesystem 100 ausgebildet, das sich insbesondere für refraktive Augenbehandlungen eignet, die der Behebung von Fehlsichtigkeiten eines zu behandelnden Auges 360 dienen. Das Laserchirurgiesystem 100 umfasst einen eine erste Laserquelle bildenden Excimer-Laser 110, der einen gepulsten Laserstrahl 140 mit einer UV-Wellenlänge erzeugt. Beispielsweise ist der Excimer-Laser ein ArF-Excimer-Laser, der bei 193 nm strahlt. Dem Excimer-Laser 110 zugeordnet ist eine Steuerelektronik 120 mit entsprechender Steuerungs- und Bedienungssoftware.

Als weitere Laserquelle enthält das Laserchirurgiesystem 100 einen Fs-Laser 130, der vorzugsweise als Faserlaser ausgebildet ist und gepulste Laserstrahlung mit Pulsdauern im Femtosekundenbereich erzeugt. Beispielsweise liegt die Pulsdauer der von dem Fs-Laser 130 erzeugten Laserpulse zwischen 100 und 800 fs; die Wellenlänge der von dem Fs-Laser 130 erzeugten Laserstrahlung liegt beispielsweise im NIR-Bereich, etwa zwischen 1020 und 1070 nm, beispielsweise 1064 nm.

Der von dem Excimer-Laser 110 erzeugte Laserstrahl 140 wird in einem Strahlrohr (Beamline) 160 geführt, das in nicht näher dargestellter Weise mit Stickstoff oder einem anderen Inertgas gespült ist. Eingebaut in das Strahlrohr 160 sind unter anderem ein passiver Umlenkspiegel 150, der den Laserstrahl 140 im gezeigten Beispielfall um einen Winkel von 90° umlenkt, ein anschließender Homogenisierer 170 zur Glättung des Strahlquerschnitts und Reduzierung etwaiger Intensitätsspitzen, eine Strahlformungsanordnung aus einer Linse 180, einer Blende 190 und einer weiteren Linse 200, um aus dem homogenisierten Laserstrahl einen definierten Teil auszuschneiden, ein hier von einem galvanometrisch gesteuerten Spiegelpaar gebildeter Scanner 210, ein weiterer passiver Umlenkspiegel 220 sowie eine Fokussierlinse 230 zur Fokussierung des Laserstrahls 140. Der Laserstrahl 140 tritt bei der Fokussierlinse 230 aus dem Strahlrohr 160 aus und wird durch ein Transmissionsfenster 240 nach außen abgegeben.

Im Strahlengang des von der Fs-Laserquelle 130 erzeugten Laserstrahls - bezeichnet mit 290 - liegen hintereinander eine Transmissionsfaser 250, eine Kompressionseinheit 260, ein passiver Umlenkspiegel 280, ein der Strahlaufweitung dienendes Teleskop (Beam Expander) 300, ein Scanner 310, ein weiterer passiver Umlenkspiegel 320 sowie ein in der Regel mehrlinsiges F-Theta-Objektiv 330 zur Fokussierung des Laserstrahls 290. Die Kompressionseinheit 260 dient zur zeitlichen Komprimierung der Laserpulse des zweiten Laserstrahls 290, die zuvor mittels eines nicht näher dargestellten, der Transmissionsfaser 250 vorgeschalteten Pulsstreckers zeitlich gestreckt und verstärkt wurden. Im Beispielfall der Fig. 1 ist die Kompressionseinheit 260 unmittelbar im Anschluss an die Transmissionsfaser 250 gezeigt. Es versteht sich, dass die Kompressionseinheit 260 auch an anderer Stelle innerhalb des an die Transmissionsfaser 250 anschließenden Teils des Strahlengangs des zweiten Laserstrahls 290 angeordnet sein kann, beispielsweise vor oder nach dem Umlenkspiegel 320. Bei der Kompressionseinheit 260 kann es sich um ein Transmissionsgitter handeln, das die mittels des erwähnten Pulsstreckers in den Pikosekundenbereich gestreckten Laserpulse auf Pulsdauern von beispielsweise 500 fs oder kürzer komprimiert. Die Kompressionseinheit 260 kann auch weggelassen werden, wenn die Transmissionsfaser 250 von einer PCF-Faser mit inhärent pulskomprimierenden Eigenschaften gebildet ist. Statt einer PCF-Faser kann auch eine LMA-Faser für die Transmissionsfaser 250 verwendet werden, freilich unter Verzicht auf eine inhärente Pulskompression innerhalb der Faser.

Der Scanner 310 kann genauso wie der Scanner 210 von einem Paar galvanometrisch betätigter Ablenkspiegel gebildet sein. Alternativ kann er beispielsweise von einem elektrooptischen Kristall gebildet sein, das bei der verwendeten Wellenlänge des zweiten Laserstrahls 290 ausreichend transmissiv ist.

Die Transmissionsfaser 250 erstreckt sich im gezeigten Beispielfall der Fig. 1 zwischen dem Fs-Laser 130 und einem weiteren Strahlrohr 270, in dem die Kompressionseinheit 260, die Umlenkspiegel 280, 320, das Teleskop 300 sowie der Scanner 310 untergebracht sind. Das Strahlrohr 270 bedarf in der Regel keiner Spülung durch ein Inertgas.

Zur Ausgestaltung des Laserchirurgiesystems 100 als Kombinationsgerät besitzt es ein Gehäuse 340 mit einem Basisteil 342 und einem oben an dem Basisteil 342 anschließenden, seitlich auskragenden Gehäusearm 344. Der Basisteil 342 des Gehäuses 340 umfasst einen Gehäuseboden 346, an dessen Unterseite vorzugsweise höhenjustierbare Stellfüße 348 angebracht sind, mittels denen das Laserchirurgiesystem 100 auf dem Fußboden oder einer anderen horizontalen Aufstellfläche aufgestellt werden kann. Der Excimer-Laser 110, dessen zugehörige Steuerelektronik 120, sowie der Fs-Laser 130 sind gemeinsam in dem Basisteil 342 des Gehäuses 340 untergebracht, wobei die beiden Laser 110, 130 in übereinander liegenden Ebenen angeordnet sind und im gezeigten Beispielfall der Excimer-Laser 110 über dem Fs-Laser 130 angeordnet ist. Die Transmissionsfaser 250 ist seitlich an dem Excimer-Laser 110 und der unter diesem angeordneten Steuerelektronik 120 vorbei nach oben geführt und mit dem Strahlrohr 270 verbunden. Dieses reicht ebenso wie das Strahlrohr 160 in den Gehäusearm 344 hinein und erstreckt sich in Armlängsrichtung bis knapp über das an dem Gehäusearm 344 gewünschtenfalls gewichtskompensiert aufgehängte Fokussierobjektiv 330 hinaus. Das Strahlrohr 160 erstreckt sich im gezeigten Beispielfall oberhalb des Strahlrohrs 270 in den Gehäusearm 344 hinein und reicht in Armlängsrichtung über das Strahlrohr 270 hinaus, so dass der aus der Fokussierlinse 230 austretende Laserstrahl 140 an dem Strahlrohr 270 vorbei zum Transmissionsfenster 240 gelangen und von dort aus dem Gehäusearm 344 austreten kann. Man erkennt, dass die Austrittsstellen der beiden Laserstrahlen 140, 290 aus dem Laserchirurgiesystem 100 in Armlängsrichtung im Abstand hintereinander liegen. Durch geringfügige seitliche Verschiebung einer unter dem Gehäusearm 344 aufgestellten Patientenliege (nicht näher dargestellt) kann somit das zu behandelnde Auge 360 des auf der Liege liegenden Patienten wahlweise unter dem Transmissionsfenster 240 oder unter dem Fokussierobjektiv 330 positioniert werden. Es versteht sich, dass alternativ das Strahlrohr 270 oberhalb des Strahlrohrs 160 in dem Gehäuse 340 angeordnet werden kann. In diesem Fall reicht das Strahlrohr 270 in Armlängsrichtung des Gehäusearms 344 über das Strahlrohr 160 hinaus; die Anordnung des Transmissionsfensters 240 und des Fokussierobjektivs 330 ist dann dementsprechend vertauscht.

Zur präzisen Referenzierung des Laserchirurgiesystems 100 gegenüber dem Auge 360 bei Durchführung einer Inzision mittels des Fs-Laserstrahls 290 ist unterseitig an dem Fokussierobjektiv 330 ein Patienteninterface 350 angebracht oder anbringbar, das seinerseits mit einem auf das Auge 360 aufgesetzten und dort durch Saugkraft festgehaltenen Saugring (nicht näher dargestellt) in Koppeleingriff bringbar ist oder selbst einen solchen Saugring aufweist.

Zur Überwachung des Operationsfortschritts und -ergebnisses verfügt das Laserchirurgiesystem 100 im gezeigten Beispielfall über zwei Mikroskope 370, 380, die am Gehäusearm 344 angebracht sind und im wesentlichen direkt über dem Fokussierobjektiv 330 bzw. dem Transmissionsfenster 240 liegen. Um eine Beobachtung des Auges 360 zu ermöglichen, sind die Umlenkspiegel 220, 320 dichroitisch ausgelegt, wobei sie für die Wellenlänge des betreffenden Laserstrahls eine hohe Reflektivität besitzen, gleichzeitig jedoch für den sichtbaren Spektralbereich stark transmissiv sind.

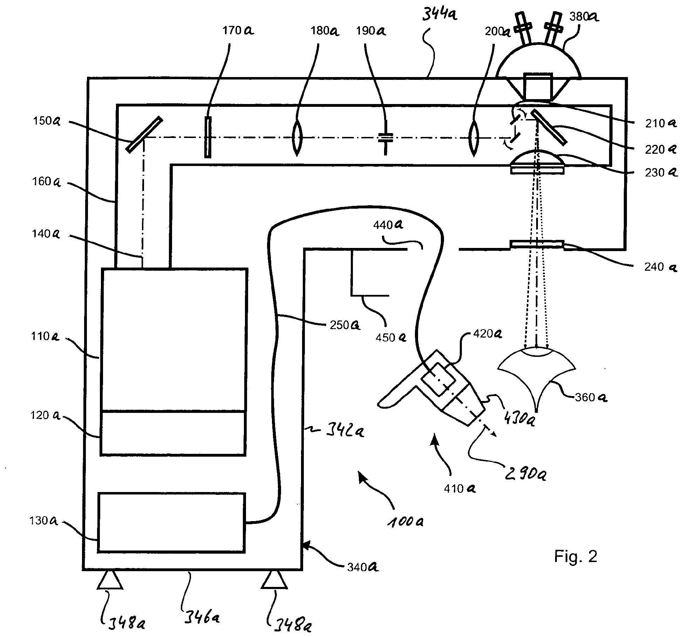

Bei der Variante gemäß Fig. 2 sind gleiche oder gleichwirkende Komponenten mit gleichen Bezugszeichen wie in Fig. 1 versehen, jedoch ergänzt durch einen Kleinbuchstaben. Zur Vermeidung unnötiger Wiederholungen wird auf die vorstehenden Erläuterungen zum Ausführungsbeispiel der Fig. 1 verwiesen, sofern sich nachstehend nichts anderes ergibt.

Das Laserchirurgiesystem 100a gemäß Fig. 2 unterscheidet sich von dem Ausführungsbeispiel der Fig. 1 vorrangig dadurch, dass die Transmissionsfaser 250a zwar an dem Excimer-Laser 110a vorbei nach oben in den Bereich des Gehäusearms 344a geführt ist, dann jedoch an einer Austrittsstelle 440a aus dem Gehäusearm 344a nach außen herausgeführt ist und in einem allgemein mit 410a bezeichneten Handstück endet. Zumindest der außerhalb des Gehäuses verlaufende Teil der Transmissionsfaser 250a ist zweckmäßigerweise in einem nicht näher dargestellten Verbindungskabel geführt, in dem zusätzlich beispielsweise noch eine Saugleitung sowie elektrische Leitungen geführt sein können. Es versteht sich, dass die verschiedenen Leitungen sowie die Transmissionsfaser 250a auf mehrere Verbindungskabel aufgeteilt sein können.

In das Handstück 410 integriert sind verschiedene optische Komponenten, welche zumindest die Funktionen der Strahlablenkung und -fokussierung erfüllen, gewünschtenfalls auch eine zeitliche Pulskompression und eine Strahlaufweitung leisten können. Die entsprechenden Komponenten sind zweckmäßigerweise als Miniaturkomponenten ausgeführt, wobei sich aus Platzgründen für die Strahlablenkung beispielsweise ein elektrooptischer Kristall eignet. Die in dem Handstück 410 untergebrachten optischen Komponenten sind in Fig. 2 schematisch durch ein Modul 420a angedeutet; selbstverständlich können sie als miniaturisierte Einzelkomponenten in das Handstück 410a integriert sein. Das Handstück 410a weist ferner ein Patienteninterface 430a auf, das der Ankopplung des Handstücks 410a an das Auge 360a oder einen auf dem Auge sitzenden Saugring dient. Neben der Anbringung intrakornealer Schnitte, insbesondere bei der LASIK, sind mit dem Handstück 410a beispielsweise auch Katarakt- oder Glaukombehandlungen als Einsatzmöglichkeiten denkbar.

Schematisch ist in Fig. 2 noch eine Ablage 450 angedeutet, auf welcher das Handstück 410a bei Nichtgebrauch abgelegt werden kann.

## Patentansprüche

1. Lasereinrichtung für die Ophthalmologie, umfassend Komponenten zur Bereitstellung eines ersten gepulsten Laserstrahls (140) mit auf die Ablation von Hornhautmaterial abgestimmten Strahleigenschaften und eines zweiten gepulsten Laserstrahls (290) mit auf die Anbringung einer Inzision im Augengewebe abgestimmten Strahleigenschaften, wobei die Komponenten gesonderte Laserquellen (110, 130) zur Erzeugung der beiden Laserstrahlen sowie eine Vielzahl optischer Elemente umfassen, welche die beiden Laserstrahlen auf gesonderten Strahlwegen zu einem jeweiligen Strahlaustrittsort führen und auf einen außerhalb des Strahlaustrittsorts liegenden Brennpunkt fokussieren,
**dadurch gekennzeichnet, dass** die optischen Elemente einen der Führung des zweiten Laserstrahls (290) dienenden Lichtwellenleiter (250) umfassen, dass zumindest die beiden Laserquellen (110, 130) in einem gemeinsamen Gehäuse (340) untergebracht sind und dass der Lichtwellenleiter zumindest auf einem Teil seiner Länge innerhalb des Gehäuses verläuft, wobei die zweite Laserquelle (130) in dem Gehäuse (340) unterhalb der ersten Laserquelle (110) angeordnet ist und der Lichtwellenleiter (250) innerhalb des Gehäuses von der zweiten Laserquelle seitlich an der ersten Laserquelle vorbei nach oben geführt ist.

2. Lasereinrichtung nach Anspruch 1, **dadurch gekennzeichnet, dass** die erste Laserquelle (110) ein Excimer-Laser und die zweite Laserquelle (130) ein Faserlaser ist.

3. Lasereinrichtung nach einem der vorhergehenden Ansprüche, **dadurch gekennzeichnet, dass** das Gehäuse (340) einen zur Aufstellung auf einer horizontalen Aufstellfläche vorgesehenen, einen Boden (346) des Gehäuses umfassenden Basisteil (342) und einen im vertikalen Abstand vom Gehäuseboden quer von dem Basisteil abstehenden Gehäusearm (344) aufweist, und dass die beiden Laserquellen (110, 130) zumindest größtenteils in dem Basisteil des Gehäuses vorzugsweise übereinander untergebracht sind und zumindest ein Teil der optischen Elemente in dem Gehäusearm untergebracht ist.

4. Lasereinrichtung nach Anspruch 3, **dadurch gekennzeichnet, dass** in den Gehäusearm (344) ein den ersten Laserstrahl (140) führendes, vorzugsweise mit einem Inertgas gespültes erstes Strahlrohr (160) eingebaut ist, dass zumindest ein Teil des Strahlwegs des zweiten Laserstrahls (290) in dem Gehäusearm außerhalb des ersten Strahlrohrs (160) verläuft und dass an dem Gehäusearm ein Austrittsfenster (240) für den ersten Laserstrahl (140) gebildet ist.

5. Lasereinrichtung nach Anspruch 4, **dadurch gekennzeichnet, dass** in den Gehäusearm (344) ein längs des ersten Strahlrohrs (160) verlaufendes zweites Strahlrohr (270) eingebaut ist, durch welches der Strahlweg des zweiten Laserstrahls (290) verläuft, und dass der Lichtwellenleiter (250) innerhalb des Gehäuses (340) zwischen der zweiten Laserquelle (130) und dem zweiten Strahlrohr (270) verläuft.

6. Lasereinrichtung nach Anspruch 5, **dadurch gekennzeichnet, dass** der Gehäusearm (344) in Armlängsrichtung im Abstand vor oder hinter dem Austrittsfenster (240) für den ersten Laserstrahl (140) eine Fokussieroptik (330) für den zweiten Laserstrahl trägt.

7. Lasereinrichtung nach Anspruch 4, **dadurch gekennzeichnet, dass** der Lichtwellenleiter (250a) aus dem Gehäuse (340a) herausgeführt ist und in einem zumindest mit einem Scanner und einer Fokussieroptik ausgestatteten Handstück (410a) zur Applikation des zweiten Laserstrahls (290a) endet, wobei das Handstück mit dem Gehäuse (340a) allein durch ein oder mehrere flexible Kabel verbunden ist, von denen eines den Lichtwellenleiter enthält.

8. Lasereinrichtung nach Anspruch 7, **dadurch gekennzeichnet, dass** der Lichtwellenleiter (250a) aus dem Gehäuse (340a) an einer Stelle (440a) herausgeführt ist, die an dem Gehäusearm (344a) in Armlängsrichtung im Abstand vor oder hinter dem Austrittsfenster (240a) für den ersten Laserstrahl (140a) liegt.

9. Lasereinrichtung nach Anspruch 7 oder 8, **dadurch gekennzeichnet, dass** das Handstück (410a) einen Pulskompressor zur zeitlichen Kompression der Pulse des zweiten Laserstrahls enthält, wobei der Pulskompressor vorzugsweise ein optisches Gitter, beispielsweise ein Transmissionsgitter, aufweist.

10. Lasereinrichtung nach einem der Ansprüche 7 bis 9, **dadurch gekennzeichnet, dass** das Handstück (410a) mit Koppelformationen (430a) ausgeführt ist, die eine mechanische Ankopplung des Handstücks an ein zu behandelndes Auge (360a) oder einen auf das Auge aufgesetzten Saugring gestatten.

11. Lasereinrichtung nach einem der vorhergehenden Ansprüche, **dadurch gekennzeichnet, dass** der Lichtwellenleiter (250) eine photonische Transmissionsfaser mit einem großen Modenfeld umfasst.

12. Lasereinrichtung nach einem der vorhergehenden Ansprüche, **dadurch gekennzeichnet, dass** zumindest ein Abschnitt des Lichtwellenleiters (250) eine zeitliche Pulskompression bewirkt.

13. Lasereinrichtung nach Anspruch 12, **dadurch gekennzeichnet, dass** zumindest der die Pulskompression bewirkende Abschnitt des Lichtwellenleiters (250) von einer photonischen Hohlkernfaser gebildet ist.

14. Lasereinrichtung nach einem der vorhergehenden Ansprüche, **dadurch gekennzeichnet, dass** die von der zweiten Laserquelle (130) erzeugten Laserpulse eine Pulsdauer im Femtosekundenbereich besitzen und zwischen die zweite Laserquelle und den Lichtwellenleiter (250) ein Pulstrecker zur zeitlichen Streckung der Laserpulse auf Pulslängen größer als 1 Pikosekunde geschaltet ist.

## Claims

1. Ophthalmic laser device, comprising components for providing a first pulsed laser beam (140) having beam properties that are tuned to ablate corneal material and a second pulsed laser beam (290) having beam properties that are tuned to apply an incision in the eye tissue, wherein the components comprise separate laser sources (110, 130) for generating the two laser beams and a large number of optical elements that guide the two laser beams, along separate beam paths, to a respective beam exit site and focus them on a focal point that lies outside the beam exit site, **characterized in that** the optical elements comprise an optical waveguide (250) which serves for guiding the second laser beam (290), **in that** at least the two laser sources (110, 130) are accommodated in a common housing (340), and **in that** the optical waveguide extends within the housing for at least some of its length, wherein the second laser source (130) is arranged in the housing (340) below the first laser source (110) and the optical waveguide (250) is guided upwardly within the housing from the second laser source laterally past the first laser source.

2. Laser device according to Claim 1, **characterized in that** the first laser source (110) is an excimer laser and the second laser source (130) is a fibre laser.

3. Laser device according to one of the preceding claims, **characterized in that** the housing (340) has a base part (342), which is provided for being placed on a horizontal placement surface and comprises a bottom (346) of the housing, and a housing arm (344), which projects transversely from the base part at a vertical distance from the housing bottom, and **in that** the two laser sources (110, 130) are accommodated at least for the main part in the base part of the housing, preferably one above the other, and at least some of the optical elements are accommodated in the housing arm.

4. Laser device according to Claim 3, **characterized in that** a first beam tube (160) is mounted in the housing arm (344), which beam tube guides the first laser beam (140) and is preferably flushed with an inert gas, **in that** at least part of the beam path of the second laser beam (290) extends within the housing arm outside the first beam tube (160), and **in that** an exit window (240) for the first laser beam (140) is formed on the housing arm.

5. Laser device according to Claim 4, **characterized in that** mounted in the housing arm (344) is a second beam tube (270), which extends along the first beam tube (160) and through which the beam path of the second laser beam (290) extends, and **in that** the optical waveguide (250) extends within the housing (340) between the second laser source (130) and the second beam tube (270).

6. Laser device according to Claim 5, **characterized in that** the housing arm (344) carries a focusing optics (330) for the second laser beam at a distance in front of or behind the exit window (240) for the first laser beam (140) in the longitudinal direction of the arm.

7. Laser device according to Claim 4, **characterized in that** the optical waveguide (250a) is guided out of the housing (340a) and terminates in a handpiece (410a), equipped at least with a scanner and a focusing optics, for the application of the second laser beam (290a), wherein the handpiece is connected to the housing (340a) merely by one or more flexible cables of which one contains the optical waveguide.

8. Laser device according to Claim 7, **characterized in that** the optical waveguide (250a) is guided out of the housing (340a) at a location (440a) that is located on the housing arm (344a) at a distance in front of or behind the exit window (240a) for the first laser beam (140a) in the longitudinal direction of the arm.

9. Laser device according to Claim 7 or 8, **characterized in that** the handpiece (410a) contains a pulse compressor for temporally compressing the pulses of the second laser beam, wherein the pulse compressor preferably has an optical grating, for example a transmission grating.

10. Laser device according to one of Claims 7 to 9, **characterized in that** the handpiece (410a) is designed with coupling formations (430a) that permit mechanical coupling of the handpiece to an eye to be treated (360a) or to a suction ring which is placed on the eye.

11. Laser device according to one of the preceding claims, **characterized in that** the optical waveguide (250) comprises a photonic transmission fibre with a large mode field.

12. Laser device according to one of the preceding claims, **characterized in that** at least a portion of the optical waveguide (250) effects a temporal pulse compression.

13. Laser device according to Claim 12, **characterized in that** at least the portion of the optical waveguide (250) that effects the pulse compression is formed by a photonic hollow-core fibre.

14. Laser device according to one of the preceding claims, **characterized in that** the laser pulses generated by the second laser source (130) exhibit a pulse duration in the femtosecond range, and a pulse stretcher for temporally stretching the laser pulses to pulse lengths of greater than 1 picosecond is connected between the second laser source and the optical waveguide (250).

## Revendications

1. Mécanisme laser pour l'ophtalmologie, comprenant des composants en vue de la production d'un premier faisceau laser (140) pulsé avec des propriétés de rayonnement ajustées à l'ablation de matière de la cornée et en vue de la production d'un deuxième faisceau laser (290) pulsé avec des propriétés de rayonnement ajustées à la pratique d'une incision dans le tissu oculaire, selon lequel les composants comprennent des sources laser (110, 130) distinctes en vue de la génération des deux faisceaux laser ainsi qu'une pluralité d'éléments optiques qui guident les deux faisceaux laser sur des trajectoires de rayonnement distinctes vers un lieu de sortie de faisceau respectif et qui les focalisent sur un point de convergence se trouvant à l'extérieur du lieu de sortie de faisceau ;
**caractérisé en ce que** les éléments optiques comprennent un guide d'onde de lumière (250) servant au guidage du deuxième faisceau laser (290), **caractérisé en ce que** tout au moins les deux sources laser (110,130) sont mises en place dans un boîtier (340) commun ; et **caractérisé en ce que** le guide d'onde de lumière se prolonge à l'intérieur du boîtier, tout au moins sur une partie de sa longueur, selon lequel la deuxième source laser (130) est disposée dans le boîtier (340), au-dessous de la première source laser (110) et le guide d'onde de lumière (250) est guidé vers le haut à l'intérieur du boîtier par la deuxième source laser, de manière latérale en passant devant la première source laser.

2. Mécanisme laser selon la revendication 1, **caractérisé en ce que** la première source laser (110) est un laser excimer et la deuxième source laser (130) est un laser à fibre.

3. Mécanisme laser selon l'une des revendications précédentes, **caractérisé en ce que** le boîtier (340) présente une partie de base (342), qui comprend un fond (346) du boîtier et qui est prévu en vue de la mise en place sur une surface de pose horizontale, et présente également un bras de boîtier (344) se trouvant en saillie à la transversale de la partie de base, dans un écartement vertical par rapport au fond du boîtier ; et **caractérisé en ce que** les deux sources laser (110, 130) sont mises en place, tout au moins pour la majeure partie, dans la partie de base du boîtier, en étant de préférence superposées, et tout au moins une partie des éléments optiques est mise en place dans le bras de boîtier (344).

4. Mécanisme laser selon la revendication 3, **caractérisé en ce qu'**un premier tube de rayonnement (160), qui est rincé de préférence au moyen d'un gaz inerte et qui guide le premier faisceau laser (140), est intégré dans le bras de boîtier (344) ; et **caractérisé en ce que** tout au moins une partie de la trajectoire de rayonnement du deuxième faisceau laser (290) se prolonge dans le bras de boîtier, à l'extérieur du premier tube de rayonnement (160) ; et **caractérisé en ce qu'**une fenêtre de sortie (240) est formée pour le premier faisceau laser (140) au niveau du bras de boîtier.

5. Mécanisme laser selon la revendication 4, **caractérisé en ce qu'**un deuxième tube de rayonnement (270), lequel se prolonge le long du premier tube de rayonnement (160), est intégré dans le bras de boîtier (344), à travers lequel deuxième tube de rayonnement (270) se prolonge la trajectoire de rayonnement du deuxième faisceau laser (290) ; et **caractérisé en ce que** le guide d'onde de lumière (250) se prolonge à l'intérieur du boîtier (340) entre la deuxième source laser (130) et le deuxième tube de rayonnement (270).

6. Mécanisme laser selon la revendication 5, **caractérisé en ce que** le bras de boîtier (344) supporte une optique de focalisation (330) pour le deuxième faisceau laser, dans la direction longitudinale du bras et se trouvant à distance à l'avant ou à l'arrière de la fenêtre de sortie (240) pour le premier faisceau laser (140).

7. Mécanisme laser selon la revendication 4, **caractérisé en ce que** le guide d'onde de lumière (250a) est guidé en dehors du boîtier (340a) et se termine dans une pièce à main (410a) pourvue tout au moins d'un scanner et d'une optique de focalisation en vue de l'application du deuxième faisceau laser (290a), selon lequel la pièce à main est seulement raccordée au boîtier (340a) par l'intermédiaire d'un ou de plusieurs câbles flexibles, dont l'un contient le guide d'onde de lumière.

8. Mécanisme laser selon la revendication 7, **caractérisé en ce que** le guide d'onde de lumière (250a) est guidé en dehors du boîtier (340a) au niveau d'un emplacement (440a) qui se situe au niveau du bras de boîtier (344a) dans la direction longitudinale du bras et se trouvant à distance à l'avant ou à l'arrière de la fenêtre de sortie (240a) pour le premier faisceau laser (140a).

9. Mécanisme laser selon la revendication 7 ou 8, **caractérisé en ce que** la pièce à main (410a) contient un compresseur à pulsations en vue de la compression dans le temps des pulsations du deuxième faisceau laser, selon lequel le compresseur à pulsations présente de préférence un réseau de diffraction optique, par exemple un réseau de transmission.

10. Mécanisme laser selon l'une des revendications 7 à 9, **caractérisé en ce que** la pièce à main (410a) est réalisée avec des formations de couplage (430a) qui permettent à la pièce à main d'être accouplée mécaniquement à un oeil (360a) devant être traité ou qui permettent de poser une bague-ventouse sur l'oeil.

11. Mécanisme laser selon l'une des revendications précédentes, **caractérisé en ce que** le guide d'onde de lumière (250) comprend une fibre de transmission photonique avec un champ modal de grande envergure.

12. Mécanisme laser selon l'une des revendications précédentes, **caractérisé en ce que** tout au moins une section du guide d'onde de lumière (250) déclenche une compression pulsée dans le temps.

13. Mécanisme laser selon la revendication 12, **caractérisé en ce que** tout au moins la section déclenchant la compression pulsée du guide d'onde de lumière (250) est formée par une fibre photonique à noyau creux.

14. Mécanisme laser selon l'une des revendications précédentes,
**caractérisé en ce que** les pulsations laser générées par la deuxième source laser (130) possèdent une durée de pulsation mesurée en femtosecondes et un dispositif d'étirement des pulsations est mis en circuit entre la deuxième source laser et le guide d'onde de lumière (250) en vue de l'étirement dans le temps des pulsations laser pour atteindre des longueurs de pulsations supérieures à 1 picoseconde.
